# EUROPÄISCHE PATENTSCHRIFT

(11) **EP 2 280 921 B1**
(45) Veröffentlichungstag und Bekanntmachung des Hinweises auf die Patenterteilung: **30.07.2014**
(21) Anmeldenummer: 09731418.1
(22) Anmeldetag: 07.04.2009
(51) Int. Cl.: C07C 51/265, C07D 307/89

(54) **VERFAHREN ZUM ANFAHREN EINES GASPHASENOXIDATIONSREAKTORS**
METHOD FOR STARTING A GAS-PHASE OXIDATION REACTOR
PROCÉDÉ DE DÉMARRAGE D'UN RÉACTEUR D'OXYDATION EN PHASE GAZEUSE

(30) Priorität: 07.04.2008 EP 08154169
(43) Veröffentlichungstag der Anmeldung: 09.02.2011
(73) Patentinhaber: BASF SE, 67056 Ludwigshafen (DE)
(72) Erfinder: WILMER, Hagen, 67071 Ludwigshafen (DE); ZÜHLKE, Jürgen, 67346 Speyer (DE); LAUTENSACK, Thomas, 69488 Birkenau (DE); ALLMANN, Hans-Martin, 74867 Neunkirchen (DE); ROSOWSKI, Frank, 68239 Mannheim (DE); DOBNER, Cornelia, Katharina, 67071 Ludwigshafen (DE)
(86) Internationale Anmeldenummer: PCT/EP2009/054168
(87) Internationale Veröffentlichungsnummer: WO 2009/124946

(56) Entgegenhaltungen:
- EP-A- 1 852 413
- DE-A1- 2 948 163
- DE-A1- 19 823 262

## Beschreibung

Die Erfindung betrifft die Herstellung von Phthalsäureanhydrid durch Gasphasenoxidation von o-Xylol. Dazu wird im Allgemeinen ein Gasstrom, der molekularen Sauerstoff und o-Xylol umfasst, durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise einer Salzschmelze, umgeben.

Trotz dieser Thermostatisierung kommt es in der Katalysatorschüttung zur Ausbildung sogenannter "Heißer Flecken" (Hot Spots), in denen eine höhere Temperatur herrscht als im übrigen Teil der Katalysatorschüttung. Diese "Hot Spots" geben Anlass zu Nebenreaktionen, wie der Totalverbrennung des Ausgangsmaterials oder führen zur Bildung unerwünschter, vom Reaktionsprodukt nicht oder nur mit viel Aufwand abtrennbarer Nebenprodukte, beispielsweise Phthalid oder Benzoesäure.

Zur Abschwächung dieser Hot Spots wurde in der Technik dazu übergegangen, unterschiedlich aktive Katalysatoren schichtweise in der Katalysatorschüttung anzuordnen, wobei in der Regel der weniger aktive Katalysator so im Festbett angeordnet ist, dass das Reaktionsgasgemisch mit ihm als erstes in Kontakt kommt, d. h. er liegt in der Schüttung zum Gaseintritt hin, wohingegen sich der aktivere Katalysator zum Gasaustritt aus der Katalysatorschüttung hin befindet (DE-OS 25 46 268, EP 286 448, DE 29 48 163, EP 163 231).

Zur Inbetriebnahme bzw. zum "Anfahren" des Reaktors wird die Katalysatorschüttung üblicherweise durch externe Beheizung auf eine Temperatur gebracht, die oberhalb der späteren Betriebstemperatur liegt. Sobald die Oxidationsreaktion in Gang kommt, wird die Reaktionstemperatur durch die ausgeprägte Exothermie der Umsetzung aufrecht erhalten und die externe Beheizung wird reduziert und schließlich abgestellt. Die Ausbildung eines ausgeprägten Hot Spots behindert allerdings eine schnelle Anfahrphase, da ab einer bestimmten Hot Spot-Temperatur der Katalysator irreversibel geschädigt werden kann. Daher wird die Beladung des Gasstroms mit dem zu oxidierenden Kohlenwasserstoff in kleinen Schritten erhöht und muss sehr sorgfältig kontrolliert werden.

Aus WO 98/00778 ist bekannt, dass der Zusatz temporärer Aktivitätsdämpfungsmittel zu einer Verkürzung der Anfahrphase führen kann.

Die EP-A-1 852 413 offenbart ein Verfahren zur Gasphasenoxidation. Ein gasförmiger Strom, der einen aromatischen Kohlenwasserstoff und molekularen Sauerstoff umfasst, wird durch wenigstens zwei Katalysatorlagen geleitet. Zwischen zwei in Strömungsrichtung des gasförmigen Stroms hintereinander angeordneten Katalysatorlagen ist eine Moderatorlage angeordnet, die katalytisch weniger aktiv als die angrenzenden Katalysatorlagen oder katalytisch inaktiv ist.

Trotz der vorgenannten Verbesserungsvorschläge sind lange Anfahrzeiten von 2 bis 8 Wochen oder länger bisher erforderlich. "Anfahrzeit" beschreibt die Zeit, die notwendig ist, um die Zufuhr des Kohlenwasserstoffs auf die gewünschte Endbeladung, d. h. die Oxidation zum stationären Zustand zu bringen, ohne den Katalysator irreversibel zu schädigen. Hierbei ist vor allem darauf zu achten, dass der Hot Spot einen gewissen kritischen Wert nicht überschreitet, da sonst die Selektivität sowie die Lebensdauer des Katalysators stark beeinträchtigt werden.

Andererseits kann die Salzbadtemperatur beim Anfahren nicht beliebig niedrig gewählt werden, da sonst im Reaktionsprodukt erhöhte Gehalte an nicht umgesetztem Kohlenwasserstoff bzw. Unteroxidationsprodukten auftreten, die zum Überschreiten von Emissions- bzw. Qualitätsvorgaben führen können.

Im Fall der technisch bedeutsamen Oxidation von o-Xylol zu Phthalsäureanhydrid beträgt die Endbeladung z. B. 80 g o-Xylol/Nm³ Luft oder mehr. Die bisher eingesetzten Katalysatoren auf Basis von Vanadiumoxid und Titandioxid werden bei Temperaturen von 360 bis 400 °C angefahren. Dadurch ist gewährleistet, dass die Rest-o-Xylolmenge sowie der Gehalt des Unteroxidationsprodukts Phthalid im Rahmen der Emissions- und Qualitätsvorgaben liegen. Im Verlauf der dann folgenden Formierungsphase wird die Salzbadtemperatur abgesenkt (auf üblicherweise etwa 350 °C) und die Beladung kann parallel dazu auf Ziellast erhöht werden.

Die WO 2005/063673 beschreibt ein Verfahren zur Herstellung ungesättigter Aldehyde und/oder Carbonsäuren durch Partialoxidation an einem Katalysatorfestbett, wobei der Reaktor eine Reaktionszone umfasst, in der als Hauptprodukt ungesättigte Aldehyde anfallen, und in dieser Reaktionszone eine Lage inaktives Material an der Stelle eingeschoben ist, an der die Lage des Hot Spots erwartet wird.

Der Erfindung liegt die Aufgabe zugrunde, ein Verfahren zum Anfahren des Gasphasenoxidationsreaktors anzugeben, das eine kurze Anfahrzeit ohne Überschreiten von Emissions- bzw. Qualitätsvorgaben, lange Katalysatorlebensdauer, hohe Ausbeute und geringe Bildung von Nebenprodukten vereint.

Die Aufgabe wird gelöst durch ein Verfahren zum Anfahren eines Gasphasenoxidationsreaktors zur Oxidation von o-Xylol zu Phthalsäureanhydrid, der wenigstens eine Katalysatorlage umfasst und mittels eines Wärmeträgermediums temperierbar ist, wobei man
a) die Katalysatorlage durch eine Moderatorlage unterbricht, die katalytisch weniger aktiv als die Katalysatorlage oder katalytisch inaktiv ist,
b) einen Gasstrom mit einer Anfangsbeladung an o-Xylol und bei einer Anfangstemperatur des Wärmetransfermediums durch den Reaktor leitet,
b) die Beladung des Gasstroms auf eine Zielbeladuhg erhöht und parallel die Temperatur des Wärmetransfermediums auf eine Betriebstemperatur senkt.

Vorzugsweise ist die Moderatorlage stromaufwärts zur Position eines zu erwartenden Hot Spots angeordnet. "Stromaufwärts zur Position eines zu erwartenden Hot Spots" bedeutet, dass sich der überwiegende Teil der räumlichen Ausdehnung der Moderatorlage stromaufwärts zur Position des Hot Spots befindet. Vorzugsweise befinden sich wenigstens 60 %, insbesondere wenigstens 80 % des Volumens der Moderatorlage stromaufwärts zur Position eines zu erwartenden Hot Spots.

Mit "Position eines zu erwartenden Hot Spots" ist die Stelle im Reaktor gemeint, an der sich in einer identischen Katalysatorschüttung unter identischen Bedingungen, wobei jedoch die Katalysatorlage nicht durch eine Moderatorlage unterbrochen ist, das höchste lokale Temperaturmaximum(maximaler Hot Spot) ausbildet. Wenn z. B. das erfindungsgemäße Verfahren zum Anfahren eines neu befüllten Reaktors angewandt wird, ist die Position des zu erwartenden Hot Spots in der Regel aus früheren Anfahrvorgängen bekannt.

Die genaue Position des maximalen Hot Spots hängt von mehreren Parametern ab, insbesondere der Eintrittstemperatur des Gases in die Katalysatorschüttung, der Aktivität des Katalysators, der Temperatur des Wärmetransfermediums, dem Gasvolumenstrom durch die Katalysatorschüttung, der o-Xylol-Beladung, dem Druck und etwaigen Temperaturinhomogenitäten im Wärmetransfermedium. Da die Position des Hot Spots daher nur schwer theoretisch vorherzusagen ist, wird diese zweckmäßigerweise mittels einer Vergleichsschüttung (ohne Moderatorlage) ermittelt, wie vorstehend ausgeführt.

Das Volumen der Moderatorlage beträgt im Allgemeinen 3 bis 25%, vorzugsweise 5 bis 10% des Volumens der Katalysatorlage, die von der Moderatorlage unterbrochen ist.

Das aus der Katalysatorlage, die von der Moderatorlage unterbrochen ist, austretende Reaktionsgemisch umfasst Phthalsäureanhydrid, Phthalsäureanhydrid-Unteroxidationsprodukte und nicht umgesetztes o-Xylol. Unter "Phthalsäureanhydrid-Unteroxidationsprodukten" werden C₈-Körper mit niedrigerer Oxidationsstufe als Phthalsäureanhydrid verstanden, die zu Phthalsäureanhydrid weiter oxidierbar sind. Hierzu zählen vor allem o-Tolylaldehyd, o-Tolylsäure und Phthalid. Phthalsäureanhydrid macht vorzugsweise mehr als 90 Mol-% der Summe von Phthalsäureanhydrid und Phthalsäureanhydrid-Unteroxidationsprodukten aus.

In bevorzugten Ausführungsformen umfasst der Reaktor wenigstens zwei in Strömungsrichtung des Gasstroms hintereinander angeordnete Katalysatorlagen unterschiedlicher Aktivität, insbesondere drei, vier oder fünf Katalysatorlagen. Die aufeinanderfolgenden Katalysatorlagen unterscheiden sich in ihrer Aktivität.

Es, sind verschiedene Konfigurationen der Aktivitätsabstufung möglich. In einer bevorzugten Ausführungsform nimmt die Aktivität der Katalysatoren in Strömungsrichtung des Gasstroms von der am nächsten zum Gaseinritt gelegenen Katalysatorlage zu der am nächsten zum Gasaustritt gelegenen Katalysatorlage von einer Katalysatorlage zur nächsten stetig zu.

Unter Aktivität eines Katalysators bzw. einer Katalysatorlage wird der Umsatz verstanden, der unter identischen Bedingungen (insbesondere hinsichtlich Katalysatorvolumen, volumenbezogener Raumgeschwindigkeit (gas hourly space velocity GHSV) bzw. Luftmenge, Temperatur des Wärmeträgermediums, Kohlenwasserstoff-Beladung des gasförmigen Stroms) in einer Testanlage gemessen wird. Je höher der Umsatz eines Katalysators bzw. einer Katalysatorlage desto höher ist dessen/deren Aktivität. Diese Methode ist insbesondere zum Vergleich von Aktivitäten bzw. zur Bestimmung relativer Katalysatoraktivitäten geeignet.

Falls der Reaktor mehr als eine Katalysatorlage umfasst, unterbricht die Moderatorlage vorzugsweise die am meisten stromaufwärts gelegene, d. h. zum Reaktoreingang gelegene, Katalysatorlage.

Im Allgemeinen regelt man die Erhöhung der Beladung des Gasstroms so, dass die Temperatur am Hot Spot in der Katalysatorlage einen vorgegebenen Grenzwert nicht überschreitet, da ab einer bestimmten Hot Spot-Temperatur der Katalysator irreversibel geschädigt werden kann, wobei die Selektivität sowie die Lebensdauer des Katalysators beeinträchtigt werden. Die Hot Spot-Temperatur kann anhand des Temperaturprofils bestimmt werden, das z. B. mittels Thermoelementen, die in Thermorohren in verschiedenen Höhen, z. B. äquidistant, angeordnet sind, oder mit einem in der Höhe verschiebbaren Thermoelement leicht bestimmt werden kann.

Bei Katalysatoren, deren katalytisch aktive Masse Vanadiumpentoxid und Titandioxid umfasst, überschreitet die Hot Spot-Temperatur vorzugsweise einen Wert von 450 °C nicht.

Man kann die Beladung des Gasstroms mit o-Xylol z. B. mit einer Rate von 0,5 bis 10 g/Nm³.Tag erhöhen.

In der Regel ist die Anfangsbeladung wenigstens 30 g/Nm³ niedriger als die Zielbeladung. Die Mindestbeladung des Gasstroms beträgt im Allgemeinen 30 g o-Xylol/ Nm³, weil die gleichmäßige Verdüsung des flüssig dosierten o-Xylols erst ab dieser Menge gewährleistet ist.

Als Zielbeladung wird die Beladung des Gasstroms mit o-Xylol im stationären Zustand, d. h. nach beendeter Anfahrphase während des produktiven Betriebs des Reaktors angesehen. Die Zielbeladung beträgt im Allgemeinen 60 bis 110 g/Nm³, meist 80 bis 100 g/Nm³.

Im Allgemeinen ist die Anfangstemperatur wenigstens 30 °C höher ist als die Betriebstemperatur, meist 35 bis 50 °C höher als die Betriebstemperatur. Unter Betriebstemperatur wird die Temperatur des Wärmeträgermediums im stationären Zustand, d. h. nach beendeter Anfahrphase während des produktiven Betriebs des Reaktors angesehen. Zur Kompensation nachlassender Katalysatoraktivität kann man im Betriebszustand die Temperatur des Wärmetransfermediums allerdings langfristig erhöhen (weniger als 10 °C/Jahr).

Im Allgemeinen beträgt die Betriebstemperatur 340 bis 365 °C, vorzugsweise 345 bis 355 °C.

Die Erfindung sieht eine Moderatorlage vor, die so angeordnet ist, dass sie vom Gasstrom, der die stromaufwärts gelegene Katalysatorschüttung verlässt, passiert wird, bevor dieser in die stromabwärts gelegene Katalysatorschüttung eintritt.

Die erfindungsgemäß vorgesehene Moderatorlage bewirkt eine Abkühlung des Gasstroms, bevor dieser in die Schüttung des stromabwärts zur Moderatorlage angeordneten Katalysators eintritt. Aufgrund der Abkühlung bildet sich im stromabwärts zur Moderatorlage gelegenen Teil der Katalysatorschüttung ein weniger ausgeprägter Hot Spot aus. daher kann beim Anfahren die Beladung des Gasstroms mit o-Xylol rascher gesteigert werden und so die Anfahrzeit verkürzt werden.

Die Moderatorlage besteht zweckmäßigerweise aus einer Schüttung eines teilchenförmigen Materials. Im Hinblick auf ein leichtes Befüllen des Reaktors und einen gleichmäßigen Druckverlust weist das teilchenförmige Material zweckmäßigerweise ähnliche Dimensionen auf wie die Katalysatorteilchen.

Die Moderatorlage kann katalytisch inaktiv sein. In diesem Fall besteht sie aus einem inerten Material, wie es z. B. auch als Katalysatorträger verwendet wird. Geeignete Trägermaterialien sind beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Die Moderatorlage kann auch Gewebe, Gestricke oder Gewirke aus Fasern oder Metalldrähten umfassen.

Die Moderatorlage kann auch katalytische Aktivität aufweisen. In diesem Fall ist die Moderatorlage weniger katalytisch aktiv als die Katalysatorschüttung, die von der Moderatorlage unterbrochen wird. Dies kann durch einen hohen Gehalt deaktivierender Zusätze, geringen Aktivmasseanteil, Verdünnung eines Katalysators mit inertem Material und/oder andere Maßnahmen, die dem Fachmann geläufig sind, erreicht werden.

Zumindest in der Katalysatorlage, die durch die erfindungsgemäß vorgesehene Moderatorlage unterbrochen wird, und vorzugsweise in allen Katalysatorlagen kommen vorzugsweise zumindest Katalysatoren zum Einsatz, deren katalytisch aktive Masse Vanadiumpentoxid (V₂O₅) und Titandioxid (vorzugsweise in der Anatas-Modifikation) umfasst. Maßnahmen zur Steuerung der Aktivität von Gasphasenoxidationskatalysatoren auf Basis von Vanadiumpentoxid und Titandioxid sind dem Fachmann an sich bekannt.

So können in der katalytisch aktiven Masse Verbindungen enthalten sein, die als Promotoren die Aktivität und Selektivität des Katalysators beeinflussen.

Als aktivitäts- bzw. selektivitätsbeeinflussende Faktoren seien beispielhaft Alkalimetallverbindungen, insbesondere Cäsiumoxid, Lithium-, Kalium-, Natrium- und Rubidiumoxid, sowie phosphor- oder schwefelhaltige Verbindungen genannt.

Eine weitere Möglichkeit der Aktivitätssteuerung besteht in der Variation des Anteils der Aktivmasse oder des V₂O₅-Gehalts am Gesamtgewicht des Katalysators, wobei höhere Aktivmassen- oder V₂O₅-Gehalte eine höhere Aktivität bedingen und umgekehrt.

Bei den im erfindungsgemäßen Verfahren verwendeten Katalysatoren handelt es sich im Allgemeinen um Schalenkatalysatoren, bei denen die katalytisch aktive Masse schalenförmig auf einem inerten Träger aufgebracht ist. Die Schichtdicke der katalytisch aktiven Masse beträgt in der Regel 0,02 bis 0,2 mm, vorzugsweise 0,05 bis 0,15 mm. Im Allgemeinen weisen die Katalysatoren eine schalenförmig aufgebrachte Aktivmasseschicht auf einem inerten Träger auf.

Als inertes Trägermaterial können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Das Trägermaterial ist in der Regel nicht-porös. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 4 bis 7 mm verwendet.

Die Aufbringung der einzelnen Schichten des Schalenkatalysators kann mit beliebigen an sich bekannten Methoden erfolgen, z. B. durch Aufsprühen von Lösungen oder Suspensionen in der Dragiertrommel oder Beschichtung mit einer Lösung oder Suspension in einer Wirbelschicht. Dabei können der katalytisch aktiven Masse organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat, Vinylacetat/Ethylen sowie Hydroxyethylcellulose zugesetzt werden, wobei mit Vorteil Bindermengen von 3 bis 20 Gew.- %, bezogen auf den Feststoffgehalt der Lösung der Aktivmassenbestandteile, eingesetzt werden. Wird die katalytisch aktive Masse ohne organische Bindemittel auf den Träger aufgetragen, so sind Beschichtungstemperaturen über 150°C von Vorteil. Bei Zusatz der oben angegebenen Bindemittel liegen die brauchbaren Beschichtungstemperaturen je nach verwendetem Bindemittel zwischen 50 und 200 °C. Die aufgetragenen Bindemittel brennen nach dem Einfüllen des Katalysators und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

In einer bevorzugten Ausführungsform des erfindungsgemäßen Verfahrens mit drei Katalysatorlagen weisen die Katalysatoren folgende Zusammensetzung auf (wobei die erste Lage die in Strömungsrichtung des Gasstroms am weitesten stromaufwärts angeordnete Lage ist):
für die erste Lage:
   7 bis 10 Gew.-% Aktivmasse bezogen auf den gesamten Katalysator, wobei diese Aktivmasse:
   6 bis 11 Gew.-% Vanadiumpentoxid
   1,2 bis 3 Gew.-% Antimontrioxid
   0,1 bis 1 Gew.-% eines Alkali (berechnet als Alkalimetall), insbesondere Cäsiumoxid enthält und als Rest auf 100 Gew.-% Titandioxid, vorzugsweise in Anatasmodifikation mit einer BET-Oberfläche von 5 bis 30 m²/g
für die zweite Lage:
   7 bis 12 Gew.-% Aktivmasse bezogen auf den gesamten Katalysator, wobei diese Aktivmasse:
   5 bis 13 Gew.-% Vanadiumpentoxid
   0 bis 3 Gew.-% Antimontrioxid
   0 bis 0,4 Gew.-% eines Alkali (berechnet als Alkalimetall), insbesondere Cäsiumoxid
   0 bis 0,4 Gew.-% Phosphorpentoxid (berechnet als P)
   enthält und als Rest zu 100 Gew.-% Titandioxid, vorzugsweise in Anatasmodifikation mit einer BET-Oberfläche von 10 bis 40 m²/g
für die dritte Lage:
   8 bis 12 Gew.-% Aktivmasse bezogen auf den gesamten Katalysator, wobei diese Aktivmasse:
   5 bis 30 Gew.-% Vanadiumpentoxid
   0 bis 3 Gew.-% Antimontrioxid
   0 bis 0,3 Gew.-% eines Alkali (berechnet als Alkalimetall), insbesondere Cäsiumoxid
   0,05 bis 0,4 Gew.-% Phosphorpentoxid (berechnet als P)
enthält und als Rest zu 100 Gew.-% Titandioxid, vorzugsweise in Anatasmodifikation mit einer BET-Oberfläche von 15 bis 50 m²/g.

Das Verhältnis der von der ersten, zweiten und dritten Lage eingenommenen Volumina beträgt vorzugsweise 100 bis 200 : 40 bis 100 : 40 bis 100.

In einer bevorzugten Ausführungsform des erfindungsgemäßen Verfahrens mit vier Katalysatorlagen weisen die Katalysatoren folgende Zusammensetzung auf (wobei die erste Lage die in Strömungsrichtung des Gasstroms am weitesten stromaufwärts angeordnete Lage ist):
für die erste Lage:
   7 bis 10 Gew.-% Aktivmasse bezogen auf den gesamten Katalysator, wobei diese Aktivmasse:
   6 bis 11 Gew.-% Vanadiumpentoxid
   1,2 bis 3 Gew.-% Antimontrioxid
   0,1 bis 1 Gew.-% eines Alkali (berechnet als Alkalimetall), insbesondere Cäsiumoxid
   enthält und als Rest zu 100 Gew.-% Titandioxid, vorzugsweise in Anatasmodifikation mit einer BET-Oberfläche von 5 bis 30 m²/g
für die zweite Lage:
   7 bis 10 Gew.-% Aktivmasse bezogen auf den gesamten Katalysator, wobei diese Aktivmasse:
   4 bis 15 Gew.-% Vanadiumpentoxid
   0 bis 3 Gew.-% Antimontrioxid
   0,1 bis 1 Gew.-% eines Alkali (berechnet als Alkalimetall), insbesondere Cäsiumoxid
   0 bis 0,4 Gew.-% Phosphorpentoxid (berechnet als P)
   enthält und als Rest zu 100 Gew.-% Titandioxid, vorzugsweise in Anatasmodifikation mit einer BET-Oberfläche von 10 bis 35 m²/g
für die dritte Lage:
   7 bis 10 Gew.-% Aktivmasse bezogen auf den gesamten Katalysator, wobei diese Aktivmasse:
   5 bis 13 Gew.-% Vanadiumpentoxid
   0 bis 3 Gew.-% Antimontrioxid
   0 bis 0,4 Gew.-% eines Alkali (berechnet als Alkalimetall), insbesondere Cäsiumoxid
   0 bis 0,4 Gew.-% Phosphorpentoxid (berechnet als P)
   enthält und als Rest zu 100 Gew.-% Titandioxid, vorzugsweise in Anatasmodifikation mit einer BET-Oberfläche von 15 bis 40 m²/g
für die vierte Lage:
   8 bis 12 Gew.-% Aktivmasse bezogen auf den gesamten Katalysator, wobei diese Aktivmasse:
   10 bis 30 Gew.-% Vanadiumpentoxid
   0 bis 3 Gew.-% Antimontrioxid
   0,05 bis 0,4 Gew.-% Phosphorpentoxid (berechnet als P)
enthält und als Rest zu 100 Gew.-% Titandioxid, vorzugsweise in Anatasmodifikation mit einer BET-Oberfläche von 15 bis 50 m²/g.

Das Verhältnis der von der ersten, zweiten, dritten und vierten Lage eingenommenen Volumina beträgt vorzugsweise 80 bis 160 : 30 bis 100 : 30 bis 100 : 30 bis 100.

Gewünschtenfalls kann man für die Phthalsäureanhydridherstellung noch einen nachgeschalteten Finishing-Reaktor vorsehen, wie er beispielsweise in der DE-A 198 07 018 oder DE-A 20 05 969 A beschrieben ist. Als Katalysator verwendet man dabei im Vergleich zum Katalysator der letzten Lage vorzugsweise einen noch aktiveren Katalysator.

Die Katalysatoren werden üblicherweise in Reaktionsrohre gefüllt, die in von außen auf die Reaktionstemperatur, beispielsweise mit einem Wärmeträgermedium, z. B. einer Salzschmelze, thermostatisiert sind. Über die so bereitete Katalysatorschüttung wird der Gasstrom bei Temperaturen von im allgemeinen 300 bis 450 °C, vorzugsweise von 320 bis 420 °C und besonders bevorzugt von 340 bis 400 °C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h⁻¹ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit o-Xylol erzeugt. Das molekularen Sauerstoff enthaltende Gas kann im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten. Vorzugsweise verwendet man Luft.

Man kann zwei oder mehr Zonen, vorzugsweise zwei Zonen der im Reaktionsrohr befindlichen Katalysatorschüttung auf unterschiedliche Reaktionstemperaturen thermostatisieren, wozu beispielsweise Reaktoren mit getrennten Salzbädern eingesetzt werden können. Vorzugsweise wird die Gasphasenoxidation ohne Aufteilung in Temperaturzonen bei einer Temperatur des Wärmeträgermediums durchgeführt. Vorzugsweise wird das Wärmeträgermedium im Gegenstrom zur Strömungsrichtung des Reaktionsgases in den Reaktionsrohren geführt. Als Temperatur des Wärmetransfermediums wird im Allgemeinen dessen Temperatur beim Eintritt in den Reaktor angesehen.

Die Erfindung wird durch die beigefügten Zeichnungen und die folgenden Beispiele näher veranschaulicht.

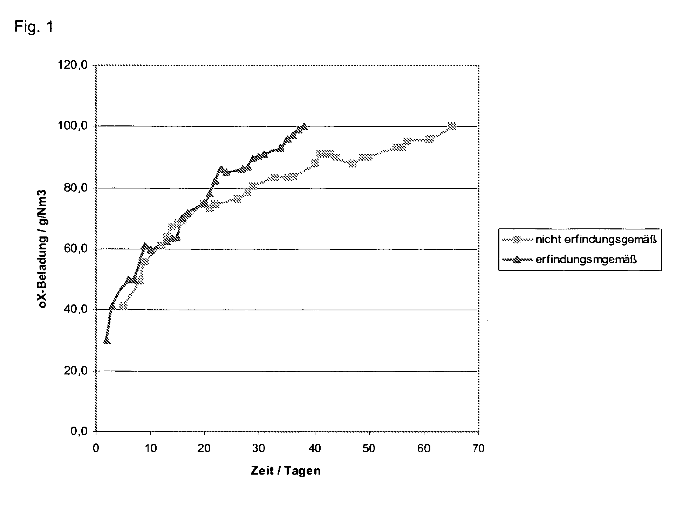

Fig. 1 zeigt den zeitlichen Verlauf (Tage) der Beladung des Gasstroms (g/Nm³) beim Anfahren eines Reaktors zur Oxidation von o-Xylol zu Phthalsäureanhydrid für ein vierlagiges Katalysatorsystem, dessen erste Lage durch eine Moderatorlage unterbrochen ist (erfindungsgemäß), und ein entsprechendes Katalysatorsystem ohne Moderatorlage (nicht erfindungsgemäß).

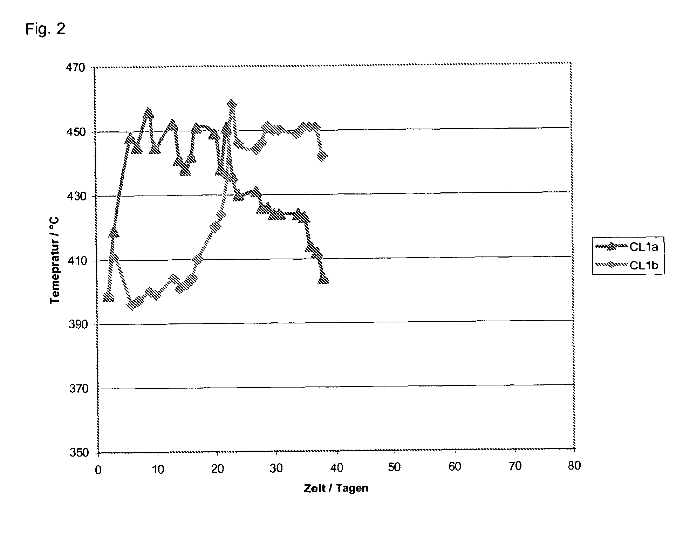

Fig. 2 zeigt den zeitlichen Verlauf der Hot Spot-Temperaturen in einer stromaufwärts zu einer Moderatorlage gelegenen Schüttung eines Katalysators 1 (CL1a) und einer stromabwärts zur Moderatorlage gelegenen Schüttung des Katalysators 1 (CL1 b).

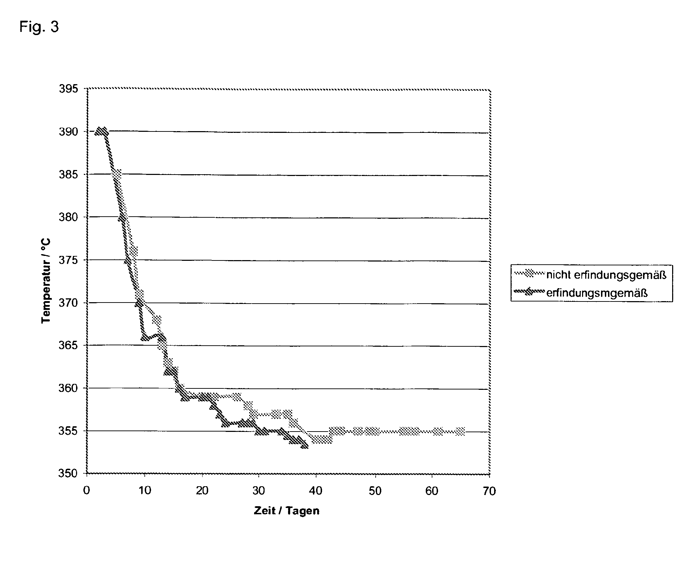

Fig. 3 zeigt den zeitlichen Verlauf der Salzbadtemperatur beim Anfahren eines Reaktors zur Oxidation von o-Xylol zu Phthalsäureanhydrid für ein vierlagiges Katalysatorsystem, dessen erste Lage durch eine Moderatorlage unterbrochen ist (erfindungsgemäß), und ein entsprechendes Katalysatorsystem ohne Moderatorlage (nicht erfindungsgemäß).

### Vergleichsbeispiel

Es wurden die folgenden Katalysatoren 1 bis 4 verwendet. Die Katalysatoren umfassten Steatitformkörper (Magnesiumsilikat) in Form von Ringen (7 x 7 x 4 mm, AD x L x ID) mit aufgebrachter Aktivmasse.

Katalysator 1: Aktivmassenanteil: 8,0 % des Gesamtgewichts des Katalysators. Aktivmassenzusammensetzung (nach Kalzination bei 400 °C für 4 h): 7,1 Gew.-% V₂O₅, 1,8 Gew.-% Sb₂O₃, 0,36 Gew.-% Cs.

Katalysator 2: Aktivmassenanteil: 8,0 % des Gesamtgewichts des Katalysators. Aktivmassenzusammensetzung (nach Kalzination bei 400 °C für 4 h): 7,1 Gew.-% V₂O₅, 2,4 Gew.-% Sb₂O₃, 0,26 Gew.-% Cs.

Katalysator 3: Aktivmassenanteil: 8,0 % des Gesamtgewichts des Katalysators. Aktivmassenzusammensetzung (nach Kalzination bei 400 °C für 4 h): 7,1 Gew.-% V₂O₅, 2,4 Gew.-% Sb₂O₃, 0,10 Gew.-% Cs.

Katalysator 4: Aktivmassenanteil: 8,0 % des Gesamtgewichts des Katalysators. Aktivmassenzusammensetzung (nach Kalzination bei 400 °C für 4 h): 20,0 Gew.-% V₂O₅, 0,38 Gew.-% P.

Als Reaktor verwendete man einen Reaktor, bestehend aus einem Eisenrohr mit einer lichten Weite von 25 mm und einer Länge von 360 cm. Das Eisenrohr war zur Temperaturregelung von einer Salzschmelze umgeben. Beginnend mit dem Katalysator 4 wurden die obigen Katalysatoren eingefüllt so dass sich folgende Schüttungslängenverteilung ergab: 130/50/70/70 cm (Katalysator 1/Katalysator 2/Katalysator 3/Katalysator 4).

Der Reaktor umfasste außerdem ein Thermorohr, das eine Temperaturmessung axial entlang der Katalysatorschüttungen erlaubte. Zu diesem Zweck enthielt das Thermorohr zusätzlich zum Katalysatorfestbett eine lediglich mit einem Temperaturmessfühler beschickte, im Thermorrohr längs desselben zentriert geführte Thermohülse (Außendurchmesser 4 mm).

Zum Betrieb der Anlage wurde ein Luft/o-Xylol-Gemisch bei einer Salzbadtemperatur von etwa 350 °C von oben nach unten durch den Hauptreaktor geleitet. Die Beladung des Luftstroms mit o-Xylol wurde im Verlauf von 60 Tagen von 30 g/Nm³ auf 100 g/nm³ gesteigert, wie durch die quadratischen Symbole in Fig. 1 gezeigt. Gleichzeitig wurde die Salzbadtemperatur von 390 °C auf 355 °C gesenkt, wie durch die quadratischen Symbole in Fig. 3 gezeigt.

### Erfindungsgemäßes Beispiel:

Das Vergleichsbeispiel wurde wiederholt, wobei jedoch nach 60 cm Schicht des Katalysators 1 eine 10 cm lange katalytisch inaktive Schicht aus Steatitringen (7 mm Aussendurchmesser, 4mm Höhe, 4 mm Innendurchmesser mit zwei Einkerbungen an den Stirnseiten) als Moderatorlage eingebracht wurde. Es ergab sich folgende Schüttungslängenverteilung: 60/10/60/50/70/70 cm (Katalysator 1a/Moderatorlage/Katalysator 1 b/Katalysator 2/Katalysator 3/Katalysator 4).

Die Beladung des Luftstroms mit o-Xylol wurde im Verlauf von 40 Tagen von 30 g/Nm³ auf 100 g/nm³ gesteigert, wie durch die dreieckigen Symbole in Fig. 1 gezeigt. Gleichzeitig wurde die Salzbadtemperatur von 390 °C auf 353 °C gesenkt, wie durch die dreieckigen Symbole in Fig. 3 gezeigt.

Fig. 2 zeigt den Verlauf der Hot Spot-Temperatur in der stromaufwärts zur Moderatorlage gelegenen Schüttung des Katalysators 1 (CL1 a) und der stromabwärts zur Moderatorlage gelegenen Schüttung des Katalysators 1 (CL1 b). Zunächst bildet sich ein Hot Spot im zum Reaktoreingang gelegenen Bereich der Schüttung CL1 a aus. Solange sich der Hot Spot in der Schüttung CL1a befindet, kann der Katalysator nicht schneller beladen werden als im Vergleichsbeispiel. Mit weiterer Absenkung der Salzbadtemperatur (Fig. 3) springt die Reaktion zunehmend später an und der Hot Spot verschiebt sich in der Katalysatorschüttung weiter stromabwärts. Es kommt zur Verlagerung des Hot Spots von der stromaufwärts zur Moderatorlage gelegenen Schüttung des Katalysators 1 (CL1 a) zur stromabwärts zur Moderatorlage gelegenen Schüttung des Katalysators 1 (CL1 b). Durch die Zwischenkühlung kann die Beladung sehr viel schneller erhöht werden. Die Zielbeladung wird schon nach etwa 40 Tagen erreicht, während es im Vergleichsbeispiel (quadratische Symbole) 60 Tage sind.

## Patentansprüche

1. Verfahren zum Anfahren eines Gasphasenoxidationsreaktors zur Oxidation von o-Xylol zu Phthalsäureanhydrid, der wenigstens eine Katalysatorlage umfasst und mittels eines Wärmeträgermediums temperierbar ist, wobei man
a) die Katalysatorlage durch eine Moderatorlage unterbricht, die katalytisch weniger aktiv als die Katalysatorlage oder katalytisch inaktiv ist,
b) einen Gasstrom mit einer Anfangsbeladung an o-Xylol und bei einer Anfangstemperatur des Wärmetransfermediums durch den Reaktor leitet,
c) die Beladung des Gasstroms auf eine Zielbeladung erhöht und parallel die Temperatur des Wärmetransfermediums auf eine Betriebstemperatur senkt.

2. Verfahren nach Anspruch 1, wobei die Moderatorlage stromaufwärts zur Position eines zu erwartenden Hot Spots angeordnet ist.

3. Verfahren nach Anspruch 1 oder 2, wobei das Volumen der Moderatorlage 3 bis 25% des Volumens der Katalysatorlage beträgt, die von der Moderatorlage unterbrochen ist.

4. Verfahren nach einem der vorhergehenden Ansprüche, wobei der Reaktor wenigstens zwei in Strömungsrichtung des Gasstroms hintereinander angeordnete Katalysatorlagen unterschiedlicher Aktivität umfasst und die Moderatorlage die am meisten stromaufwärts gelegene Katalysatorlage unterbricht.

5. Verfahren nach einem der vorhergehenden Ansprüche, wobei man die Erhöhung der Beladung des Gasstroms so regelt, dass die Temperatur am Hot Spot in der Katalysatorlage einen vorgegebenen Grenzwert nicht überschreitet.

6. Verfahren nach Anspruch 5, wobei der Grenzwert der Temperatur am Hot Spot 450 °C beträgt.

7. Verfahren nach einem der vorhergehenden Ansprüche, wobei die Anfangsbeladung wenigstens 30 g/Nm³ niedriger ist als die Zielbeladung.

8. Verfahren nach einem der vorhergehenden Ansprüche, wobei die Anfangstemperatur wenigstens 30 °C höher ist als die Betriebstemperatur.

9. Verfahren nach einem der vorhergehenden Ansprüche, wobei die Zielbeladung 60 bis 110 g/Nm³ beträgt.

10. Verfahren nach einem der vorhergehenden Ansprüche, wobei die Betriebstemperatur 340 bis 365 °C beträgt

11. Verfahren nach einem der vorhergehenden Ansprüche, wobei man die Beladung mit einer Rate von 0,5 bis 10 g/Nm³.Tag erhöht.

12. Verfahren nach einem der vorhergehenden Ansprüche, wobei die Katalysatorlage einen Katalysator enthält, dessen katalytisch aktive Masse Vanadiumpentoxid und Titandioxid umfasst.

## Claims

1. A process for starting up a gas phase oxidation reactor for oxidation of oxylene to phthalic anhydride, said reactor comprising at least one catalyst layer and being temperature-controllable by means of a heat carrier medium, wherein
a) the catalyst layer is interrupted by a moderator layer which is less catalytically active than the catalyst layer or is catalytically inactive,
b) a gas stream is passed through the reactor with an initial loading of oxylene and at an initial temperature of the heat transfer medium,
c) the loading of the gas stream is increased to a target loading and, in parallel, the temperature of the heat transfer medium is lowered to an operating temperature.

2. The process according to claim 1, wherein the moderator layer is disposed upstream of the position of an expected hotspot.

3. The process according to claim 1 or 2, wherein the volume of the moderator layer is from 3 to 25% of the volume of the catalyst layer which is interrupted by the moderator layer.

4. The process according to any one of the preceding claims, wherein the reactor comprises at least two catalyst layers of different activity arranged in succession in flow direction of the gas stream and the moderator layer interrupts the catalyst layer furthest upstream.

5. The process according to any one of the preceding claims, wherein the increase in the loading of the gas stream is regulated such that the temperature at the hotspot in the catalyst layer does not exceed a predefined limit.

6. The process according to claim 5, wherein the limit in the temperature at the hotspot is 450°C.

7. The process according to any one of the preceding claims, wherein the initial loading is at least 30 g/m³ (STP) lower than the target loading.

8. The process according to any one of the preceding claims, wherein the initial temperature is at least 30°C higher than the operating temperature.

9. The process according to any one of the preceding claims, wherein the target loading is from 60 to 110 g/m³ (STP).

10. The process according to any one of the preceding claims, wherein the operating temperature is from 340 to 365°C.

11. The process according to any one of the preceding claims, wherein the loading is increased at a rate of from 0.5 to 10 g/m³ (STP).day.

12. The process according to any one of the preceding claims, wherein the catalyst layer comprises a catalyst whose catalytically active material comprises vanadium pentoxide and titanium dioxide.

## Revendications

1. Procédé pour démarrer un réacteur d'oxydation en phase gazeuse en vue de l'oxydation d'o-xylène en anhydride de l'acide phtalique, qui comprend au moins une couche de catalyseur et qui peut être équilibré thermiquement au moyen d'un agent caloporteur, dans lequel
a) on interrompt la couche de catalyseur par une couche de modérateur, qui est catalytiquement moins active que la couche de catalyseur ou qui est catalytiquement inactive,
b) on guide un flux de gaz contenant une charge de départ d'o-xylène et à une température de départ de l'agent de transfert de chaleur au travers du réacteur,
c) on augmente la charge du flux de gaz à une charge cible et on abaisse parallèlement la température de l'agent de transfert de chaleur à une température d'exploitation.

2. Procédé selon la revendication 1, la couche de modérateur étant disposée en amont de la position d'un point chaud attendu.

3. Procédé selon la revendication 1 ou 2, le volume de la couche de modérateur représentant 3 à 25% du volume de la couche de catalyseur qui est interrompue par la couche de modérateur.

4. Procédé selon l'une quelconque des revendications précédentes, le réacteur comprenant au moins deux couches de catalyseur disposées les unes derrière les autres dans le sens d'écoulement du flux gazeux, d'activité différente et la couche de modérateur interrompant la couche de catalyseur située le plus en amont.

5. Procédé selon l'une quelconque des revendications précédentes, l'augmentation de la charge du flux de gaz étant réglée de manière telle que la température au point chaud dans la couche de catalyseur ne passe pas au-dessus d'une valeur limite prédéfinie.

6. Procédé selon la revendication 5, la valeur limite de la température au point chaud étant de 450°C.

7. Procédé selon l'une quelconque des revendications précédentes, la charge de départ étant inférieure d'au moins 30 g/Nm³ à la charge cible.

8. Procédé selon l'une quelconque des revendications précédentes, la température de départ étant supérieure d'au moins 30°C à la température d'exploitation.

9. Procédé selon l'une quelconque des revendications précédentes, la charge cible étant de 60 à 110 g/Nm³.

10. Procédé selon l'une quelconque des revendications précédentes, la température d'exploitation étant de 340 à 365°C.

11. Procédé selon l'une quelconque des revendications précédentes, la charge étant augmentée d'un taux de 0,5 à 10 g/Nm³.jour.

12. Procédé selon l'une quelconque des revendications précédentes, la couche de catalyseur contenant un catalyseur dont la masse catalytiquement active comprend du pentoxyde de vanadium et du dioxyde de titane.
